# EUROPEAN PATENT APPLICATION

(11) **EP 2 520 565 A1**
(43) Date of publication of application: **07.11.2012**
(21) Application number: 11164684.0
(22) Date of filing: 03.05.2011
(51) Int. Cl.: C07C 215/24, C07F 9/38, C07D 263/06, C07D 309/12

(54) **Stereoselective Synthesis of cis-4-Methylsphingosin**

(71) Applicant: Humboldt Universität zu Berlin, 10099 Berlin (DE)
(72) Inventor: Arenz, Christoph, 10249 Berlin (DE); Proksch, Denny, 12557 Berlin (DE)
(74) Representative: Schulz Junghans

(57) **Abstract**

The invention provides for synthesis of a (S-R) dihydroxy-amino-methylalkylene characterized by the general formula I, or its R-S diastereomer, wherein R1 is CH₂OH or CH₂-CH₂OH, and R2 is C₂ to C₂₀ alkyl, whereby a compound characterized by the general formula II, with R3, R4 and R5 being protecting groups for amino and hydroxyl functions, respectively, is used as a starting material. The invention further provides novel intermediates for syntheses of, and analogues to, cis-4-methylsphingosine.

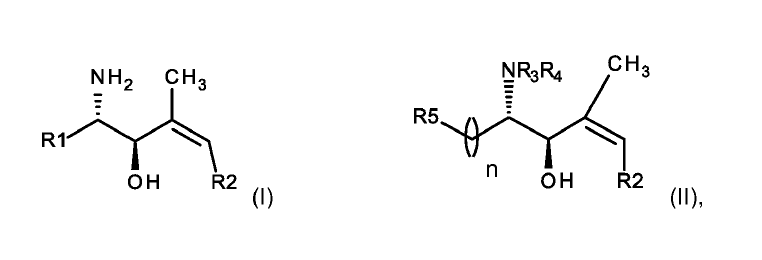

## Description

The present invention relates to the synthesis of cis-4-methylsphingosine and derivatives thereof, as well as to tert-butyl (*4S*)-4-[(Z,1R)-1-hydroxy-2-methyl-hexadec-2-enyl]-2,2-dimethyl-oxazolidine-3-carboxylate and tert-butyl N-[(*Z*,*1S*,*2R*)-2-hydroxy-3-methyl-1-(tetrahydropyran-2-yloxymethyl)heptadec-3-enyl]carbamate as starting materials or syntheses intermediates. It further relates to (*Z,3S,4R*)-3-amino-5-methyl-nonadec-5-ene-1,4-diol and [(Z,3S,4R)-3-amino-4-hydroxy-5-methyl-nonadec-5-enyl]phosphonic acid.

Sphingosine-1-phosphate is a known lipid messenger molecule and an agonist of specific G-protein coupled receptors S1 P1 to S1 P5. These receptors play an important role in cardiovascular processes, angiogenesis and immune responses.

The compound FTY720 (Fingolimod) is a sphingosine analogue phosphorylated in biological systems to form FTY720-phosphate and that influences S1 P-receptors. The exact mode of action of the compound is currently unknown; nevertheless the compound was recently approved for treatment of multiple sclerosis in the United States and Russia.

Sphingosine analogue Cis-4-methylsphingosin was synthesised by R. R. Schmidt and coworkers in 1993. Similar to FTY720 this compound is phosphorylated in biological systems and inhibits the activity of different S1 P-receptors. The spectrum of receptors modulated by Cis-4-methylsphingosine is slightly different to that modulated by FTY720. Additionally, cis-4-methylsphingosine kills neuroblastoma cells.

Following the method of Schmidt, synthesis of Cis-4-methylsphingosine is performed in 9 sequential steps, starting with D-galactose. Cis-4-methylsphingosine is synthesised through a challenging sequence of protection, deprotection and modification steps. The synthesis yield is only 0.3%. An inversion of the configuration of the C2 atom is possible through inversion of the reaction introducing the azide group, which later becomes the amino group. The optical rotation value ad20=-3.6° (c=1) in chloroform may be indicative of this fact. Furthermore, the time required for a well trained experimenter to perform the synthesis is at least 3 weeks, more likely 3 months.

Various groups working in the field have found it difficult to synthesise the compound using the method described. Poor characterisation of the compound may be due also to its scarcity. Because the compound may support the activity of FTY720, there is pharmaceutical interest in and demand for cis-4-methylsphingosin.

The objective of the present invention is to provide a simple and economical means for preparation of Cis-4-methylsphingosine.

In the present invention, a stereoselective synthesis of cis-4-methylsphingosine is reduced to two steps starting from easily accessible starting material, and the overall yield attained is raised to approx. 30 % compared to the synthesis known in the art. This facilitates further characterisation of the substance. Shortened and simplified Cis-4-methylsphingosin synthesis will make cis-4-methylsphingosine more readily available.

According to a first aspect of the invention, a method for preparation of a (S-R) dihydroxy-amino-methylalkylene characterized by the general formula I 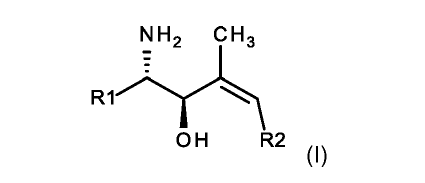 wherein
R1 is CH₂OH or CH₂-CH₂OH, and
R2 is C₂ to C₂₀ alkyl, or aryl-substituted C₂ to C₂₀ alkyl, is provided. According to this aspect, the starting material for the synthesis of I is a compound characterized by the general formula II 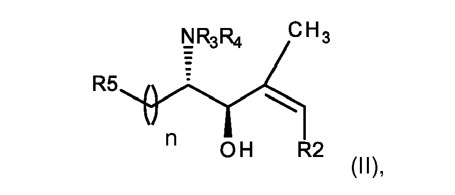 wherein R3, R4 and R5 have the following meaning:
R5 is
- OPO₃²⁻, CH₂PO₃²⁻, O-THP(tetrahydropyranyl ether), O-Bn (benzyl ether), O-TBDMS (*tert*-butyldimethylsilyl ether) or O-MOM (methoxymethyl ether) and n is 1 or 2; or
- O-trityl (triphenylmethyl ether), MMT (methoxytriphenylmethyl) and n is 2, and R3 is H and R4 is BOC (tert-butoxycarbonyl), or R3 and R4 are part of a phtalimide group, or
R5 and R3 together form a propylidene and R4 is H.

While the formulae above show the (2S, 3R) diastereomer (or the (3S, 4R) diastereomer in case of n=2), the (2R, 3S) or (3R, 4S) diastereomer can be obtained similarly by starting with the corresponding chiral educt.

In the context of the instant invention, a propylidene is molecule with the formula CH₃-C-CH₃, wherein the central carbon atom is substituted by two methyl groups and bridges the amino function and the hydroxyl function of R5 in formula II.

"Alkyl" herein may be a branched or unbranchened hydrocarbon chain. R2 in its broadest sense may be an alkyl of 2 to 20 carbon atoms in length, or an aryl-substituted alkyl. An aryl is a cyclic aromatic hydrocarbon.

For R2, a chain of C₅ to C₁₈ alkyl is preferred. Even more preferred is C₈ to C₁₅ alkyl, with C₁₃H₂₈ being the most preferred embodiment. The alkyl may be saturated or unsaturated, with saturated alkyl being preferred, unsubstituted saturated alkyl being most preferred.

According to another embodiment, the alkyl of R2 (as in the definition given in the previous paragraph) may be substituted by functional groups such as halogen, substituted or unsubstituted amine(s), nitro, cyano, isocyano, thiocyano or isothiocyano groups, alcohol or ether or ester groups or their sulfur analogues. Examples for functional groups are -Br, -CI, - CHO, -COOH, -OH, -HSO₃, -NH₂, -NO₂, and -SO₂NH₂.

An aryl-substituted alkyl according to the first aspect of the invention may be a branched or unbranched hydrocarbon chain of 2 to 20 carbon atoms in length which is substituted by aryls, preferably 5 to 18 carbon atoms, more preferred 8 to 15 carbon atoms. The aromatic ring may be composed of hetero atoms such as sulfur, oxygen or nitrogen. The aryl may also be substituted by functional groups as described in the preceding paragraph.

According to another preferred embodiment of the first aspect of the invention, a method for preparation of a S-R-dihydroxy-amino-methylalkylen or its R-S diastereomer is provided, wherein R2 is C₁₃H₂₈ and the product of synthesis is (*2S*,3*R*,4Z)- or (2*R*,3*S*,4Z)-2-amino-4-methyl-octadec-4-ene-1,3-diol or (Z,*3S*,*4R*)- or (Z,*3R,4S*)-3-amino-5-methyl-nonadec-5-ene-1,4-diol.

According to another preferred embodiment, a method for preparation of a S-R-dihydroxy-amino-methylalkylen I or its R-S diastereomer is provided, wherein the compound characterized by the general formula II is converted in the presence of a strong organic acid. Trifluoroacetic acid or a similar protic acid is preferred. The reaction is preferably performed in an aprotic, polar solvent, for example dichloroethane. Additionally, the conversion reaction is best performed at low temperatures, most preferably at 0 °C.

According to yet another embodiment of the first aspect of the invention, a method for preparation of a S-R-dihydroxy-amino-methylalkylen I or its R-S diastereomer is provided, wherein the compound characterized by the general formula II is
a) *tert*-butyl (4S)-4-[(Z,1R)-1-hydroxy-2-methyl-hexadec-2-enyl]-2,2-dimethyl-oxazolidine-3-carboxylate III 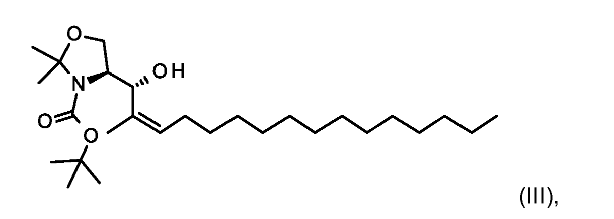 or its diastereomer *tert*-butyl (*4R*)-4-[(Z,1S)-1-hydroxy-2-methyl-hexadec-2-enyl]-2,2-dimethyl-oxazolidine-3-carboxylate:, or
b) tert-butyl N-[(*Z*,*1S,2R*)-2-hydroxy-3-methyl-1-(tetrahydropyran-2-yloxymethyl)heptadec-3-enyl]carbamate IV 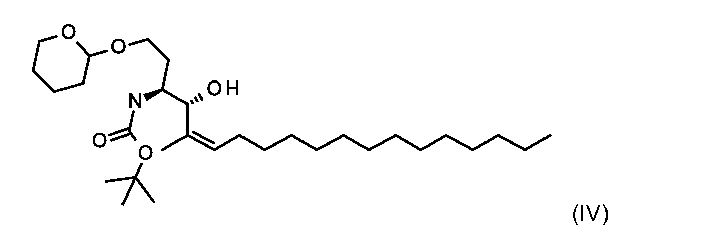 or its diastereomer *tert*-butyl N-[(Z,*1R,2S*)-2-hydroxy-3-methyl-1-(tetrahydropyran-2-yloxymethyl)heptadec-3-enyl]carbamate.

According to another embodiment of the first aspect of the invention, a method for preparation of a S-R-dihydroxy-amino-methylalkylen I or its R-S diastereomer is provided. Thererein, a vinyl halide characterized by the general formula Va 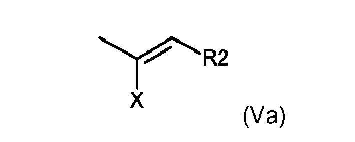 wherein R2 has the meaning specified above and X can be CI, Br or I
is used as educt to prepare the starting material according to the first aspect of the invention. According to a preferred embodiment, a vinyl bromide characterized by the general formula V, 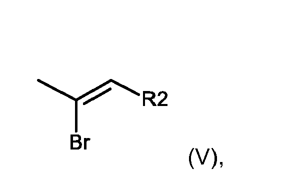 is used as an educt to prepare the starting material (characterized by formula II above) for the synthesis of I. A most preferred vinyl bromide is (Z)-2-bromohexadec-2-ene VI.

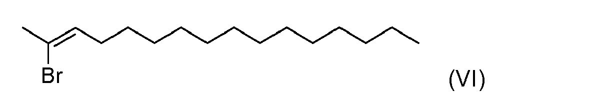

According to another embodiment, a method for preparation of a S-R-dihydroxy-amino-methylalkylen I or its R-S diastereomer is provided, wherein the vinyl bromide is prepared from a 2-ketoalkyl compound characterized by the general formula VII.

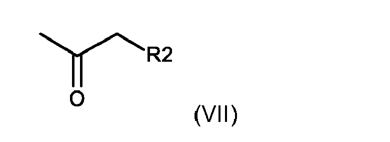

A preferred alternative of this embodiment starts from hexadecan-2-one, which is converted to (Z)-2-bromohexadec-2-ene.

According to yet another embodiment, a method for preparation of a S-R-dihydroxy-amino-methylalkylen I or its R-S diastereomer is provided, wherein a protected homoserine derivative of the general formula VIII 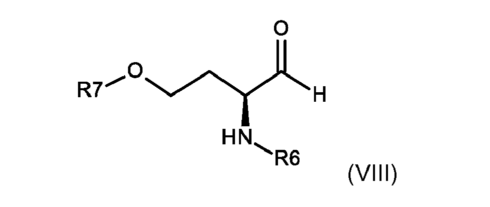 wherein
R6 is H or a amino protecting group, and
R7 is H, OPO₃²⁻, THP, Bn (benzyl), TBDMS, MOM, trityl or MMT, is reacted with the vinyl bromide.

The term amino protecting group in the sense of the present invention refers to a functional group that can be easily attached to and removed from amino groups under mild chemical conditions. Such amino protecting groups are, without being restricted to, BOC, Fmoc (fluorenylmethyloxicarbonyl), benzyloxycarbonyl or allyloxycarbonyl.

According to another embodiment, a method for preparation of a S-R-dihydroxy-amino-methylalkylen I or its R-S diastereomer is provided, wherein the protected homoserine derivative is tert-butyl N-[(*1S*)-1-formyl-3-tetrahydropyran-2-yloxy-propyl]carbamate IX 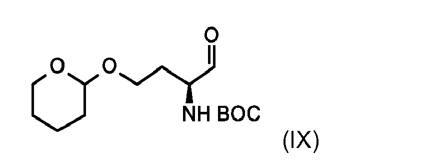 and IX is obtained by reduction of benzyl (*2S*)-2-(*tert*-butoxycarbonylamino)-4-tetrahydropyran-2-yloxy-butanoate X

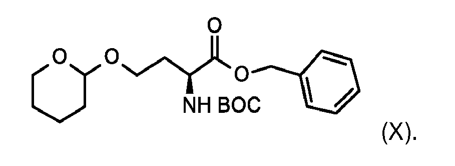

According to yet another preferred embodiment, the method for preparation of a S-R-dihydroxy-amino-methylalkylen I or its R-S diastereomer is preceded by a step, in which benzyl (*2S*)-2-(*tert*-butoxycarbonylamino)-4-hydroxy-butanoate or its diastereomer 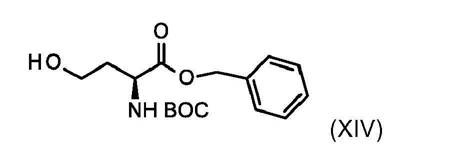 is used as starting material in a reaction to obtain benzyl (*2S*)-2-(tert-butoxycarbonylamino)-4-tetrahydropyran-2-yloxy-butanoate X.

According to yet another embodiment, the method of the previous paragraph may be preceded by a step, wherein (*2S*)-2-(tert-butoxycarbonylamino)-4-hydroxy-butanoic acid 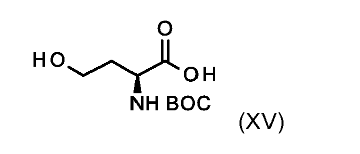 is converted to benzyl (*2S*)-2-(*tert*-butoxycarbonylamino)-4-hydroxy-butanoate XIV.

XV may be obtained by protecting the amino function of homoserine with the BOC group.

According another aspect of the invention, products of a method of synthesis according to the first aspect of the invention, or derivatives thereof, are provided. These products can be used in assays or as candidate compounds for pharmaceutical development, for example as substitutes for sphingosine or as agonists or antagonists for sphingosine receptors. The compounds thus provided are
(*Z,3S,4R*)-3-amino-5-methyl-nonadec-5-ene-1,4-diol XI 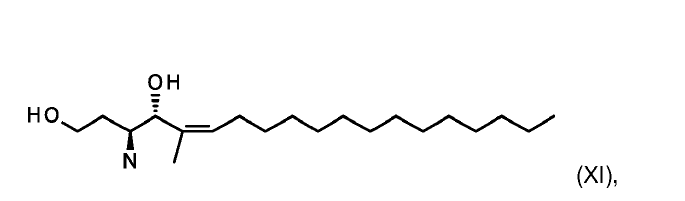 [(Z,3S,4R)-3-amino-4-hydroxy-5-methyl-nonadec-5-enyl] dihydrogen phosphate XVI 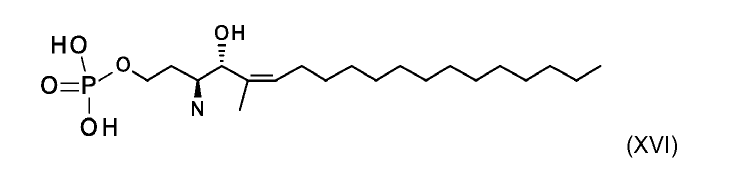 and

[(Z,*3S*,*4R*)-3-amino-4-hydroxy-5-methyl-nonadec-5-enyl]phosphonic acid XII

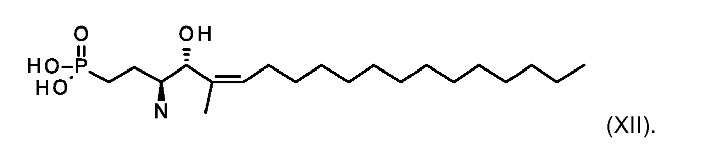

According to another aspect of the invention, the following intermediates of a method of synthesis according to first aspect of the invention are provided:
*-tert*-butyl (*4S*)-4-[(Z,*1R*)-1-hydroxy-2-methyl-hexadec-2-enyl]-2,2-dimethyl-oxazolidine-3-carboxylate XIII 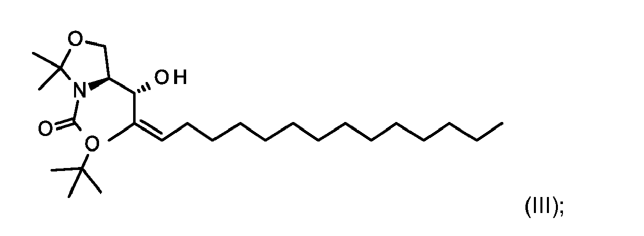
*tert*-butyl N-[(Z,*1S*,*2R*)-2-hydroxy-3-methyl-1-(tetrahydropyran-2-yloxymethyl)heptadec-3-enyl]carbamate XIV 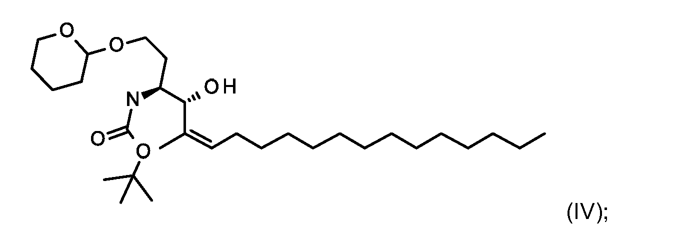
(Z)-2-Bromohexadec-2-ene VI 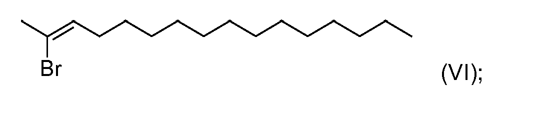 *tert*-butyl N-[(1S)-1-formyl-3-tetrahydropyran-2-yloxy-propyl]carbamate: IX 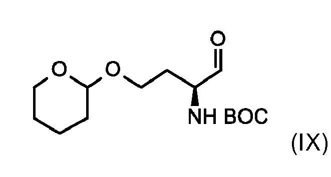 and benzyl (2S)-2-(*tert*-butoxycarbonylamino)-4-tetrahydropyran-2-yloxy-butanoate X

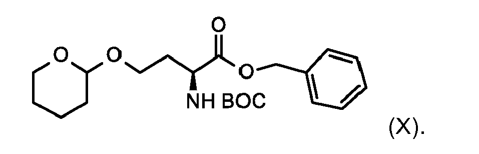

According to another aspect of the invention, a method for the synthesis of an intermediate or starting material for a method of synthesis according to the first aspect of the invention is provided, wherein the compound characterized by the general formula II, wherein R2, R3, R4 and R5 have the meaning specified above, is obtained by reacting an vinyl bromide of general formula V with an aldehyde compound characterized by the general formula XIII, wherein n, R3, R4 and R5 have the meaning specified above.

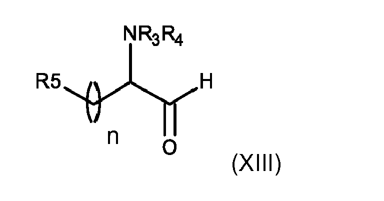

According to another aspect of the invention, a method for the synthesis of an intermediate or starting material for a method of synthesis according to the first aspect of the invention is provided, wherein tert-butyl N-[(*1S*)-1-formyl-3-tetrahydropyran-2-yloxy-propyl]carbamate IX is obtained by reduction of benzyl (*2S*)-2-(*tert*-butoxycarbonylamino)-4-tetrahydropyran-2-yloxy-butanoate X.

The synthesis described in the present invention enables production of cis-4-methylsphingosine in 3 to 5 days starting from commercially available substances Garneraldehyde and hexadecanone. Synthesis yield is 27.6 %, a 100-fold increase compared to the synthesis described by Schmidt. Significant time and cost savings are the result. Intermediates (Z)-2-Bromohexadecanone and (S)-tert-Butyl-4-((R,Z)-1-hydroxy-2-methylhexadec-2-enyl)-2,2-dimethyloxazolidin-3-carboxylate are novel compounds, not yet described in the literature. In contrast , formation of Z-alkenes from 2-ketones is known. Also, use of Lithium-vinyl compounds and Vinyl-Grignard-compounds are known and described in detail. Intermediates are characterised by ¹H-NMR, ¹³C-NMR, IR, optical rotation value and high-resolution-MS for the first time. Synthesis is simple, fast and cost-effective. Addition derivates of Cis-4-methylsphingosin like compound 5 (?) or metabolically stable phosphate-analogues can be made with the new synthesis sequence.

### Description of the figures

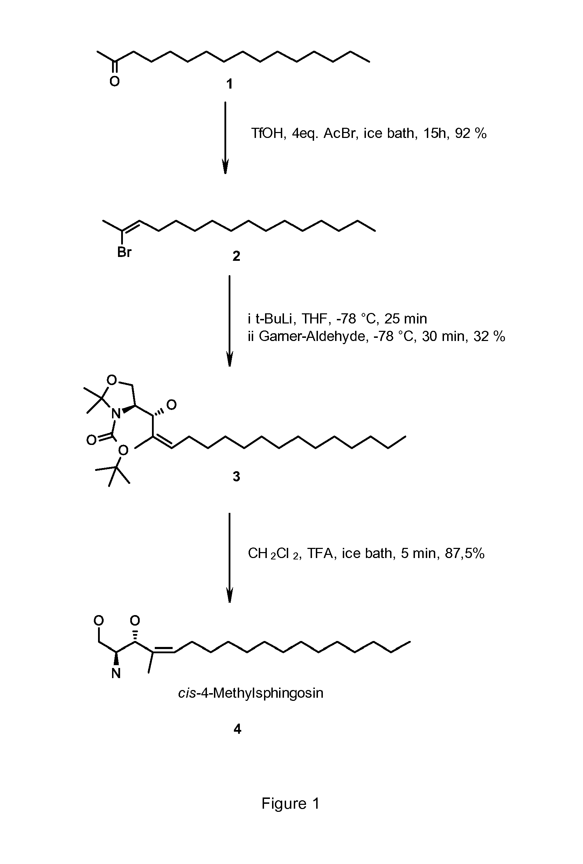

- Figure 1: shows the reaction schema of of (2S,3R,4Z)-2-Amino-4-methyl-4-octadecene-1,3-diol synthesis.

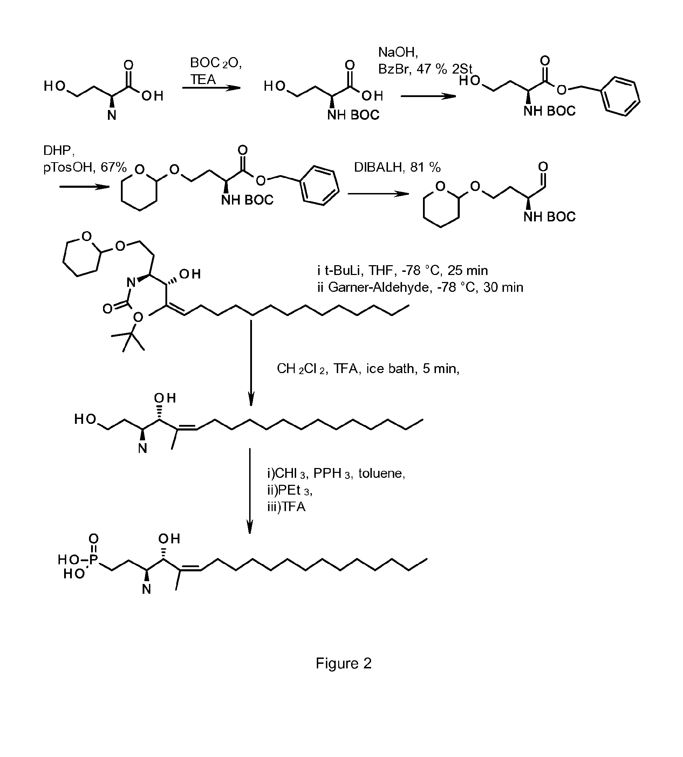

- Figure 2: shows the reaction schema of of (Z,3S,4R,5Z)-3-amino-5-methyl-nonadec-5-ene-1,4-diol synthesis.

### Examples

Synthesis of (Z)-2-Bromohexadec-2-ene

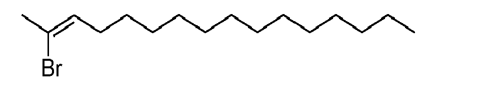

To an ice cooled solution of 200 mg (0.834 mmol) hexadecan-2-one () in 3 ml methanesulfonic acid 248 µl acetylbromid (3.336 mmol, 4 eq.) was added drop wise. Stirring was continued for 5 h in an ice bath and then 10 h at RT. The mixture was poured into 50 ml ice cooled aqueous 10 % NaOH solution and extracted with 30 ml ethyl acetate. The organic layer was washed with 50 ml saturated NaC solution and dried over Na₂SO₄. The solvent was removed to give 260 mg slightly yellow oil as crude product, which was purified by chromatography on silica gel eluted with cyclohexane. Yield: 228 mg (0.755 mmol, 90.5 % d. Th.) colorless liguid, C₁₆H₃₁Br (302.5 g/mol).
R_{f} (Cyclohexane/ethyl acetate 4:1) = 0.75
¹H-NMR (CDCl₃; 500.1 MHz): δ = 0.88 (t, *J₁*=6.9 Hz, *J₂*=6.9 Hz, 3H, CH₃) 1.25-1.28 (m, 20H), 1.37 (m, 2H, CH₂-5), 2.12 (dd, 2H, *J₁*=6.9 Hz, *J₂*=14.6 Hz, CH₂-4), 2.27 (d, *J*=1.3 Hz, 3H, CH₃-1), 5.60 (td, *J₁*=1.3 Hz, *J₂*=6.9 Hz, 1H, CH) ppm.
¹³C-NMR (CDCl₃; 125.8 MHz): δ = 14.1, 22.7, 28.5, 28.8, 29.2, 29.4, 29.5, 29.6, 29.7, 31.5, 32.0, 41.2, 53.0, 122.1, 129.2 ppm.
IR (neat) u (cm⁻¹) = 2955, 2922, 2852, 1549, 564, 549, 541, 527.
HRMS EI+ calculated for C₁₆H₃₁⁷⁹Br m/z = 302.1609, found 302.1609.
HRMS EI+ calculated for C₁₆H₃₁⁸¹Br m/z = 304.1589, found 304.1591.

### Synthesis of (S)-tert-Butyl 4-((R, Z)-1-hydroxy-2-methylhexadec-2-enyl)-2,2-dimethyloxazolidine-3-carboxylate

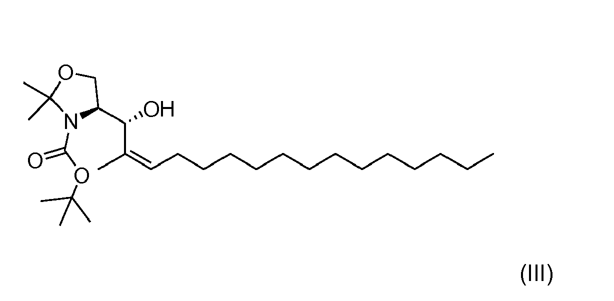

To a stirred solution of 50 mg (0.16 mmol) (Z)-2-Bromohexadec-2-ene in 5 ml THF at -78°C 0.1 ml t-BuLi-Solution (1.7 M in pentane) was added drop wise After 3 min 100 µl TMEDA were added. Stirring was continued for 3 min at -78°C. A solution of 38 mg (0.16 mmol) Garneraldehyde in 5 ml THF was added drop wise and stirring continued for 8 min. The mixture was poured into 10 ml ice cooled water and extracted three times with 10 ml Ethyl acetate. The combined organic layer was washed with 20 ml saturated NaCl-Solution and dried over Na₂SO₄. Solvent was removed to give 60 mg slightly yellow oil as crude product, which was purified by chromatography on silica gel eluted with Cyclohexane/Ethyl acetate 9:1-3:1. Yield: 228 mg (0.755 mmol, 31.7% d. Th.) colorless liquid, C₁₆H₃₁Br (302.5 g/mol).
**R**_{f} (Cyclohexane/Ethyl acetate 5.5:1.5) = 0.27
[α]_{D}²⁰ = - 43.8° (c = 1.00; CHCl₃)
**¹H-NMR** (CDCl₃; 500.1 MHz): δ = 0.83 (t, *J₁*=6.9 Hz, J₂=6.9 Hz, 3H, CH₃) 1.20 (s, 22H), 1.37-1.47 (m, 15H, BOC, 2 CH₃), 1.73 (s, 3H, CH₃-4), 1.95-2.00 (m, 1H, CH₂-6), 2.04-2.08 (m, 1H, CH₂-6), 3.89 (bs, 1H, OH), 4.01-4.07 (m, 2H, CH₂-1), 4.22 (bd, 1H, CH-2), 4.62 (d, 1H, *J*=5.0 Hz, CH₂-3), 5.24 (t, *J₁*=7.1 Hz, *J₂*=7.1 Hz, 1H, CH-5) ppm.
**¹³C-NMR** (CDCl₃; 125.8 MHz): δ = 14.1, 22.7, 26.9, 28.0, 28.4, 29.4, 29.6, 29.6, 29.7, 31.9, 64.5, 70.2, 80.4, 129.9 ppm.
IR (neat) u (cm⁻¹) = 3006, 2980, 2940, 2883, 1694, 1392, 1383, 1366, 1253, 1173, 1097.
**HRMS EI+** calculated for C₂₇H₅₁N₁O₄ m/z = 453.3818, found 453.3818.

Synthesis of (2S, 3R, 4Z)-2-Amino-4-methyl-4-octadecene-1,3-diol.

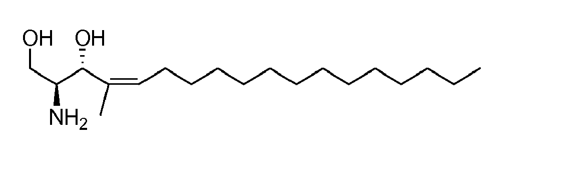

To an ice cooled solution of 125 mg (0.276 mmol) (S)-*tert*-Butyl-4-((*R,Z*)-1-hydroxy-2-methylhexadec-2-enyl)-2,2-dimethyloxazolidine-3-carboxylate in 10ml CH₂Cl₂ was added 1 ml trifluoroacetic acid drop wise. Stirring was continued for 5 min in an ice bath. The mixture was poured into 50 ml ice cooled 10 % NaOH solution and extracted with 30 ml CH₂Cl₂. The organic layer was washed with 50 ml saturated NaCl-Solution and dried over Na₂SO₄. Solvent was removed to give 93 mg colorless oil as crude product, which was purified by chromatography on silica gel eluted with cyclohexane/ethyl acetate 4:1-1:1. Yield: 75 mg (0.755 mmol, 87.2 % d. Th.) colorless solid, C₁₉H₃₉NO₂ (313.8 g/mol).
**R**_{f} (cyclohexane/ethyl acetate 1:1) = 0.27
[α]D²⁰ = - 18.8° (c = 1.00; CHCl₃)
**¹H-NMR** (CDCl₃; 500.1 MHz): δ = 0.88 (t, *J₁*=6.9 Hz, *J₂*=6.9 Hz, 3H, CH₃) 1.25 (s, 22H), 1.76 (s, 3H, CH₃-4), 1.94-2.01 (m, 1H, CH₂-6), 2.02-2.12 (m, 1H, CH₂-6), 2.27 (bs, 4H, 2 OH, NH₂,), 3.60 (br, 1H, CH-2), 3.80 (dd, 1H, *J₁*=2.8 Hz, *J₂*=11.3 Hz, CH₂-1a), 3.96 (dd, 1H, *J₁*=3.6 Hz, *J₂*=11.3 Hz, CH₂-1 b) 4.68 (d, 1H, J=7.4 Hz, CH₂-3), 5.36 (t, *J₁*=7.3 Hz, *J₂*=7.3 Hz, 1H, CH-5) ppm.
**¹³C-NMR** (CDCl₃; 125.8 MHz): δ = 14.1, 18.3, 22.7, 27.6, 28.3, 28.4, 29.4, 29.6, 29.6, 29.7, 29.7, 30.0, 31.9, 54.2, 63.5, 70.7, 130.5, 133.6 ppm.
IR (neat) u (cm⁻¹) = 3386, 2955, 2917, 2849, 1691, 1663, 1527, 1469, 1294, 1249, 1165, 1055, 1027, 997.
**MS ESI+** calculated for C₁₉H₃₉NO₂+H⁺ m/z = 314.306, found 314.311.
**MS ESI+** calculated for C₁₉H₃₉NO₂+Na⁺ m/z = 336.288, found 336.296.
**HRMS EI+** calculated for C₁₉H₃₉NO₂ m/z = 313.2981, found 313.2981.

Synthesis of (S)-2 (*tert*-Butyloxycarbonylamino)-4-hydroxybutyric acid sodium salt 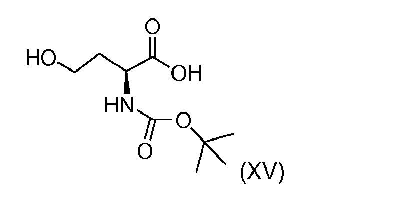 1.0 g (8.4 mmol) homoserine was suspended in 10 ml Dioxane and 15 ml water. 1.77 g (21 mmol) NaHCO₃ was added. The solution was cooled to -15 °C and a solution of 2 g (9.2 mmol) Di-tert-butyl dicarbonate in 5 ml Dioxane added drop wise Stirring was continued for 12 h at RT. 1 M HCI-Solution was added until the pH-value became acidic. It was extracted 3 times with 50 ml ethyl acetate. Combined organic layers were washed with 50 ml saturated NaHCO₃-Solution and dried over Na₂SO₄. The solvent was removed to give 1.8 g (8.2 mmol, 97.6 % d. Th.) colorless solid (C₉H₁₇NO₅ (219.2 g/mol)) as crude product, which was used without further purification.
R_{f} (ethyl acetate/methanol 98:2) = 0.24
[α]D²⁰ = - 24.9° (c = 1.00; Methanol)
¹H-NMR (CDCl₃; 500,1 MHz): δ = 1.45 (s, 10H, BOC, OH), 2.19 (m, 1H, CH₂-3), 2.75 (br m, 1H, CH₂-3), 4.24 (ddd, *J₁*=5.9 Hz, *J₂*=9.3 Hz, *J₃*=11.4 Hz, 1H, CH₂-4), 4.24 (br s, 1H, CH₂-4), 4.43 (t, *J₁*=9.0 Hz, *J₂*=9.0 Hz, 1H, CH-2), 5.10 (bs, 1H, NH) ppm.
¹³C-NMR (CDCl₃; 125 MHz): δ = 28.2, 30.6, 50.2, 65.7, 80.6, 155.4, 175.3 ppm.
MS ESI+ calculated for C₉H₁₆NO₅ m/z = 218.1, found 218.1.

### Synthesis of (S) - 2 (tert-Butyloxycarbonylamino)-4-hydroxybutyric acid benzylester

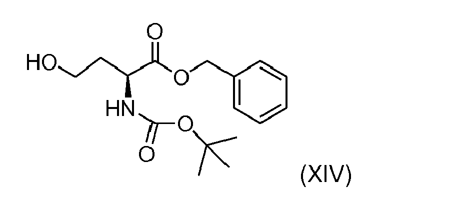

The crude product (8.4 mmol) of the BOC-homoserine protection was converted to the Sodium salt with NaOH (8.5 mmol) in 10 ml H₂O and 15 ml ethanol. The salt was suspended in 10 ml Dimethylformamide and 1.00 ml (1.44 g, 8.5 mmol) benzylbromide added. The solution was stirred for 12 h at RT. 1 M HCl-Solution was added until the pH-value became acidic. This was extracted 3 times with 20 ml ethyl acetate. Combined organic layers were washed with 30 ml saturated NaHCO₃-Solution and dried over Na₂SO₄. The solvent was removed under vacuum. The crude product was purified by chromatography on silica gel eluted with cyclohexane/ ethyl acetate 3:1-1:1. Yield: 2.04 g (6.6 mmol, 80,6 %) colorless solid, C₁₆H₂₃NO₅ (309,1 g/mol). Crude product was used without further purification.
R_{f} (Cycolhexane/Ethyl Acetate 3:1) = 0.15
[α]D²⁰ = + 5.9° (c = 1.00; Chloroform)
¹H-NMR (CDCl₃; 500,1 MHz): δ = 1.43-1.45 (s, 9H), 2.16 (m, 1H, CH₂-3), 2.30 (br m, 1H, CH₂-3), 4.24 (ddd, *J₁*=5.9 Hz, *J₂*=9.3 Hz, *J₃*=11.4 Hz, 1H, CH₂-4), 4.34 (br s, 1H, CH₂-4), 4.43 (t, *J₁*=8.8 Hz, *J₂*=8.8 Hz, 1H, CH-2), 4.69 (s, 2H, CH₂-phenyl), 5.18 (d, *J₁*=3.7 Hz, 1H), 7.33 (m, 5H, phenyl) ppm.
¹³C-NMR (CDCl₃; 125 MHz): δ = 28.3, 30.7, 36.2, 50.7, 58.3, 65.4, 65.8, 67.3, 80.5, 127.0, 127.6, 128.3, 128.6, 128.6, 140.9, 172.7 ppm.
HRMS ESI+ calculated for C₁₆H₂₃N₁O₅+H⁺ m/z = 310.1649, found 310.1650.

### Synthesis of tert-Butyl (S)-1-((benzyloxy)carbonyl)-3-(tetrahydro-2H-pyran-2-yloxy)propylcarbamate

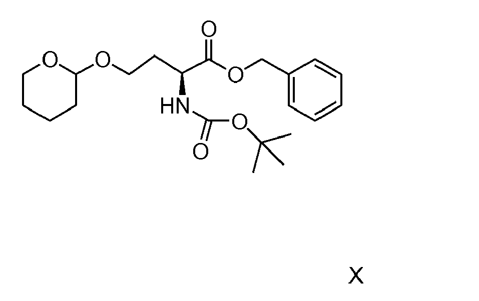

2.04 mg (6.6 mmol) of BOC-homoserinebenzylester was dissolved in 10 ml toluene. 800 µl Dihydropyran and a catalytic amount of p-TosOH were subsequently added. The reaction mixture was stirred for 45 min at RT. Saturated NaHCO₃-Solutin was added and the solution extracted twice with 50 ml Ethyl Acetate. Combined organic layers were washed with 30 ml saturated NaCl-Solution and dried over Na₂SO₄. After the solvent was removed under reduced pressure, the crude product was purified by chromatography on silica gel eluted with Cyclohexane/ Ethyl Acetate 9:1-4:1. Yield: 2.04 g (5.09 mmol, 76 %) colorless oil, C₂₁H₃₁NO₆ (393.2 g/mol).
R_{f} (Cycolhexane/Ethyl Acetate 3:1) = 0.36
[α]_{D}²⁰ = - 10.2° (c = 1.00; chloroform)
¹H-NMR (CDCl₃; 500.1 MHz): δ = 1.43-1.45 (s, 9H, BOC), 1.52-1.60 (m, 4H), 1.67-1.72 (m, 1H), 1.78-1.84 (m, 1H), 2.03-2.11 (m, 1H), 2.12-2.19 (m, 1H), 3.40-3.52 (m, 2H), 3.79-3.88 (m, 2H), 4.48 (dd, *J₁*=6.4, *J₂*=11.8 Hz, 1H), 4.55 (td, *J₁*=3.4, *J₂*=3.4, *J₃*=19.4 Hz, 1H), 5.16
(dd, *J₁*=1-9, *J₂*=12.2 Hz, 1 H), 5.21 (dd, *J₁*=5.2, *J₂*=12.3 Hz, 1 H), 5.57 (dd, *J₁*=7.7, *J₂*=21.7 Hz, 1 H), 7.33-38 (m, 5H, phenyl) ppm.
¹³C-NMR (CDCl₃; 125 MHz): δ = 19.2, 25.4, 26.3, 28.3, 30.4, 31.5, 31.8, 52.0, 61.7, 62.1, 63.6, 63.9, 66.9, 79.6, 99.0, 128.2, 128.3, 128.6, 135.5, 155.4, 172.4 ppm.
HRMS ESI+ calculated for C₂₁H₃₁NO₆+H⁺ m/z = 394.2224, found 394.2226.

### Synthesis of tert-Butyl (S)-1-formyl-3-(tetrahydro-2H-pyran-2-yloxy)propylcarbamate

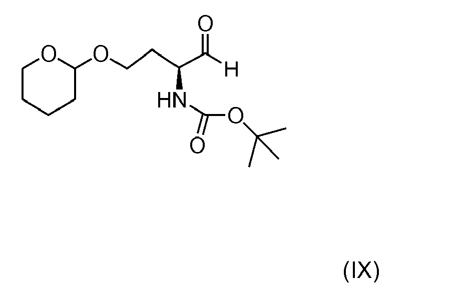

A solution of 2.04 g (5.09 mmol) tert-butyl (S)-1-((benzyloxy)carbonyl)-3-(tetrahydro-2H-pyran-2-yloxy)propylcarbamate in 30 ml toluene was cooled to -78 °C. 8.5 ml DIBALH-Solution 1.2 M in toluene were added drop wise The reaction mixture was stirred for 2 h at - 78 °C. 5 ml methanol was added at -78 °C. The reaction mixture was allowed to warm to RT. Water was added and the reaction mixture was extracted twice with 30 ml Ethyl Acetate. Combined organic layers were washed with 30 ml saturated NaCl-Solution and dried over Na₂SO₄. Subsequently the solvent was removed under reduced pressure, the crude product was purified by chromatography on silica gel eluted with Cyclohexane/ Ethyl Acetate 9:1-4:1. Yield: 1.25 g (4.34 mmol, 85.6 %) colorless oil, C₁₄H₂₅NO₅ (287.3 g/mol).
R_{f} (Cycolhexane/Ethyl Acetate 3:1) = 0.20
[α]_{D}²⁰ = + 25.8° (c = 1.00; Chloroform)
¹H-NMR (CDCl₃; 500.1 MHz): δ = 1.44 (s, 9H, BOC), 1.48-1.57 (m, 4H), 1.62-1.68 (m, 1H), 1.72-1.78 (m, 1H), 2.07-2.23 (m, 2H), 3.41-3.51 (m, 2H), 3.72-3.86 (m, 2H), 4.28 (m, 1H), 4.52 (td, *J₁*=3.0, *J₂*=3.0, *J₃*=7.0 Hz, 1H), 5.56 (dd, *J₁*=4.4, *J₂*=39.7 Hz, 1 H), 9.59 (s, 1H, aldehyde) ppm.
¹³C-NMR (CDCl₃; 125 MHz): δ = 19.2, 19.3, 25.3, 28.3, 29.3, 29.5, 30.5, 62.3, 63.0, 98.9, 155.7, 199.3 ppm.
HRMS ESI+ calculated for C₁₄H₂₅NO₅+H⁺ m/z = 288.1805, found 288.1806.

## Claims

1. Method for preparation of a (S-R) dihydroxy-amino-methylalkylene **characterized by** the general formula I 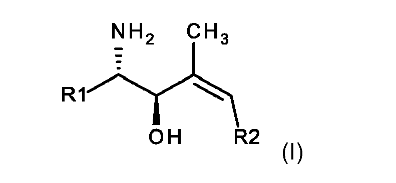 or its R-S diastereomer, wherein
- R1 is CH₂OH or CH₂-CH₂OH, and
- R2 is C₂ to C₂₀ alkyl, or aryl-substituted C₂ to C₂₀ alkyl,
whereby a compound **characterized by** the general formula II 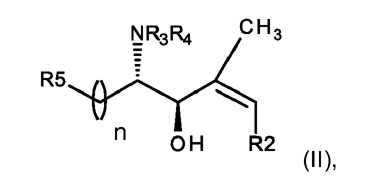 or its R-S diastereomer, wherein R3, R4 and R5 have the following meaning:
R5 is
- OPO₃²⁻, CH₂PO₃²⁻*,* O-THP, O-Bn, O-TBDMS or O-MOM and n is 1 or 2; or
- O-trityl, MMT and n is 2, and
R3 is H and R4 is BOC, or R3 and R4 are part of a phtalimid group, or
R5 and R3 together form a propylidene and R4 is H,
is used as a starting material.

2. Method according to claim 1, wherein R2 is C₁₃H₂₈ and the product is (2S,3R,4Z)- or (2R,3S,4Z)-2-Amino-4-methyl-octadec-4-ene-1,3-diol or (Z,3S,4R)- or(*Z*,*3R*,*4S*)-3-amino-5-methyl-nonadec-5-ene-1,4-diol.

3. Method according to claim 1 or 2, wherein the starting material is converted in the presence of a strong organic acid, preferably trifluoroacetic acid.

4. Method according to any of the claims 1 to 3, wherein the starting material is
a) *tert*-butyl (4S)-4-[(*Z,1R*)-1-hydroxy-2-methyl-hexadec-2-enyl]-2,2-dimethyl-oxazolidine-3-carboxylate III 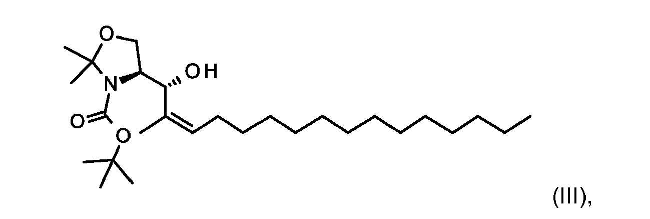 or its diastereomer tert-butyl (*4R*)-4-[(*Z,1S*)-1-hydroxy-2-methyl-hexadec-2-enyl]-2,2-dimethyl-oxazolidine-3-carboxylate:, or
b) tert-butyl N-[(*Z,1S,2R*)-2-hydroxy-3-methyl-1-(tetrahydropyran-2-yloxymethyl)heptadec-3-enyl]carbamate IV: 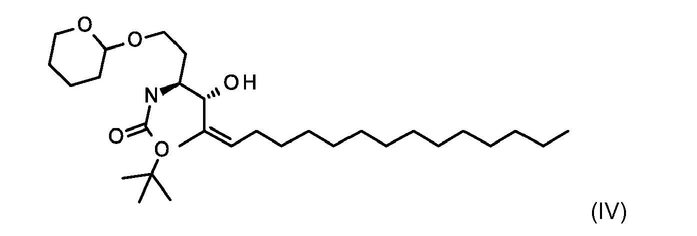 or its diastereomer tert-butyl N-[(*Z,1R,2S*)-2-hydroxy-3-methyl-1-(tetrahydropyran-2-yloxymethyl)heptadec-3-enyl]carbamate.

5. Method according to any of the claims 1 to 4, wherein a vinyl bromide **characterized by** the general formula V, 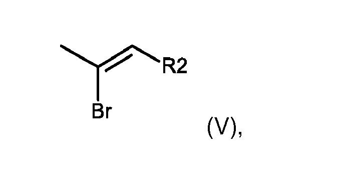 preferably (Z)-2-Bromohexadec-2-ene VI 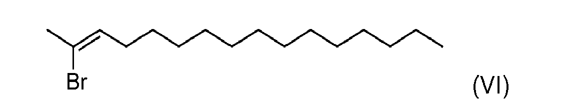 is used as educt to prepare the starting material.

6. Method according to claim 5, wherein the vinyl bromide is prepared starting from an 2-ketoalkyl compound **characterized by** the general formula VII

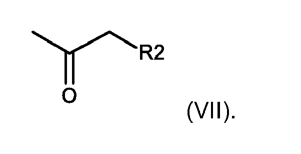

7. Method according to any of the claims 5 to 6, wherein a protected homoserine derivative of the general formula VIII 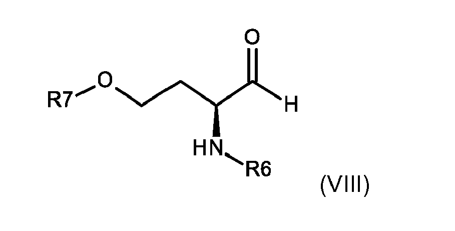 wherein
- R6 is H or a amino protecting group, and
- R7 is H, OPO₃²⁻, THP, Bn, TBDMS, MOM, trityl or MMT,
is reacted with the vinyl bromide.

8. Method according to claim 7, wherein the protected homoserine derivative is tert-butyl N-[(*1S*)-1-formyl-3-tetrahydropyran-2-yloxy-propyl]carbamate: IX: 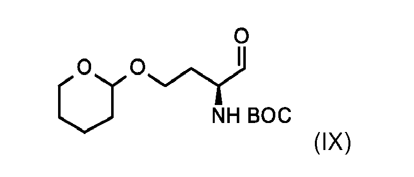 and IX is obtained by reduction of benzyl (*2S*)-2-(tert-butoxycarbonylamino)-4-tetrahydropyran-2-yloxy-butanoate X:

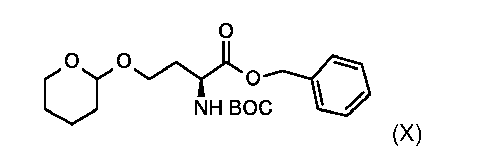

9. (Z,3S,4R)-3-amino-5-methyl-nonadec-5-ene-1,4-diol XI: 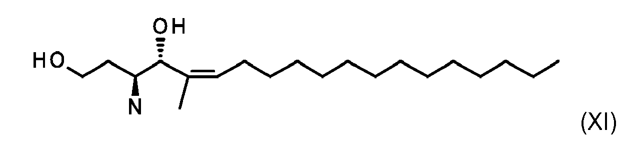 or [(*Z,3S,4R*)-3-amino-4-hydroxy-5-methyl-nonadec-5-enyl]phosphonic acid XII:

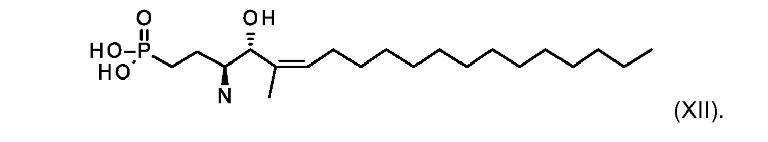

10. The following intermediates of a method of synthesis according to claim 1 to 8: -*tert*-butyl (*4S*)-4-[(*Z,1R*)-1-hydroxy-2-methyl-hexadec-2-enyl]-2,2-dimethyl-oxazolidine-3-carboxylate XIII: 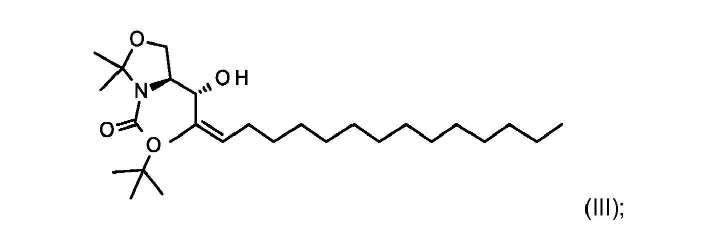 *tert*-butyl N-[(*Z,1S,2R*)-2-hydroxy-3-methyl-1-(tetrahydropyran-2-yloxymethyl)heptadec-3-enyl]carbamate XIV: 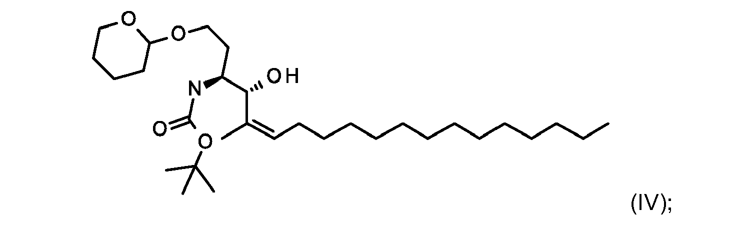 (Z)-2-Bromohexadec-2-ene VI 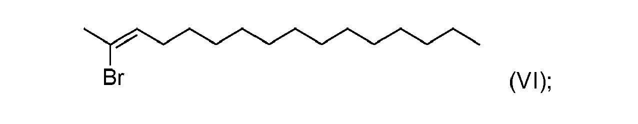 *tert-*butyl N-[(*1S*)-1-formyl-3-tetrahydropyran-2-yloxy-propyl]carbamate: IX: 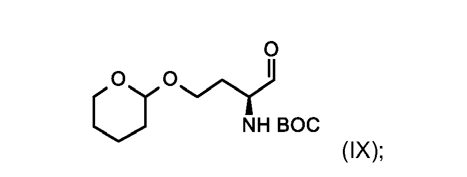 benzyl (*2S*)-2-(*tert*-butoxycarbonylamino)-4-tetrahydropyran-2-yloxy-butanoate X:

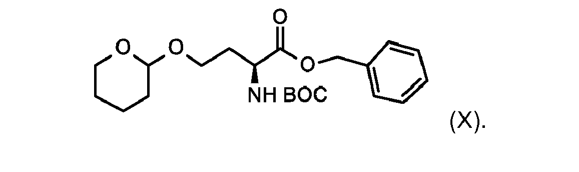

11. A method for the synthesis of an intermediate or starting material for a method of synthesis according to claims 1 to 0, wherein the compound **characterized by** the general formula II -wherein R2, R3, R4 and R5 have the meaning specified above, is obtained by reacting an vinyl bromide of general formula V with an aldehyde compound **characterized by** the general formula XIII, wherein n, R3, R4 and R5 have the meaning specified above.

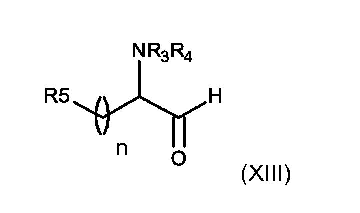

12. A method for the synthesis of an intermediate or starting material for a method of synthesis according to claims 5 to 0, wherein tert-butyl N-[(*1S*)-1-formyl-3-tetrahydropyran-2-yloxy-propyl]carbamate: IX is obtained by reduction of benzyl (2S)-2-(tert-butoxycarbonylamino)-4-tetrahydropyran-2-yloxy-butanoate X.
